# EUROPEAN PATENT APPLICATION

(11) **EP 4 545 640 A1**
(43) Date of publication of application: **30.04.2025**
(21) Application number: 23205612.7
(22) Date of filing: 24.10.2023
(51) Int. Cl.: C12N 15/113, A61P 35/00, C07K 16/18

(54) **A COMBINATION OF A LIVIN INHIBITOR AND AN INHIBITOR OF THE INTERACTION BETWEEN PD-1 AND ITS LIGAND PD-L1 OR THEIR INTRACELLULAR DOWNSTREAM SIGNALING PATHWAYS**

(71) Applicant: King Faisal Specialist Hospital & Research Centre, Riyadh, 11211 (SA)
(72) Inventor: GHEBEH, Hazem, 11211 Riyadh (SA); ALAIYA, Ayodele, 11211 Riyadh (SA); ELFOLY, Marwa, 11211 Riyadh (SA)
(74) Representative: Engelhard, Markus

(57) **Abstract**

The present invention relates to a combination of a Livin inhibitor and an inhibitor of the interaction between PD-1 and its ligand PD-L1, or a combination of a Livin inhibitor and an inhibitor of the intracellular downstream signaling pathway of a complex formed by PD-1 and PD-L1 binding, or a combination of a Livin inhibitor and an inhibitor of the intracellular downstream signaling pathway of PD-1 or PD-L1.

## Description

### FIELD OF THE INVENTION

The present invention relates to a combination of a Livin inhibitor and an inhibitor of the interaction between PD-1 and its ligand PD-L1, or a combination of a Livin inhibitor and an inhibitor of the intracellular downstream signaling pathway of a complex formed by PD-1 and PD-L1 binding, or a combination of a Livin inhibitor and an inhibitor of the intracellular downstream signaling pathway of PD-1 or PD-L1.

### BACKGROUND OF THE INVENTION

Cancer is the second leading cause of death globally, accounting for an estimated 9.6 million deaths, or one in six deaths, in 2018. While breast, colorectal, lung, cervical and thyroid cancer are the most common among women, lung, prostate, colorectal, stomach, and liver cancer are the most common among men.

The global growth of the cancer burden poses a challenge for health care systems and exerts tremendous physical, emotional, and financial strain on individuals, families, and communities. In many low- and middle-income countries, health care systems are poorly prepared to manage this burden, and many cancer patients lack access to timely and highquality diagnosis and treatment. In countries with strong health care systems, accessible early detection, quality treatment and survivorship care helped to improve the survival rates of many types of cancers. However, for aggressive or therapy-resistant forms of cancer the prognosis remains poor.

Breast cancer (BC) is the most commonly diagnosed type of cancer in women. Uncontrolled proliferation is one of the cardinal features of cancer cells. Cells with a higher rate of proliferation eventually form aggressive tumors, leading to disease progression, especially when coupled with features of metastasis. The accelerated cancer cell proliferation usually results from a dysregulation in the cell cycle and proliferation-related signaling such as PI3K/AKT, MAPK/ERK, and Wnt/β-catenin pathways.

Recently, the approval of immune checkpoint inhibitors (ICPIs), pembrolizumab, and atezolizumab has provided promising options for BC treatment. ICPIs block immune inhibitory molecules such as Programmed cell death protein 1 (PD-1) or its ligand Programmed death-ligand 1 (PD-L1), thereby invigorating the suppressed anticancer immune response.

However, PD-L1 plays an intrinsic role in promoting tumor progression, independent of its well-established immunomodulatory effects in various tumor types. Importantly, ICPIs have minimal efficacy as monotherapy and, thus, should be used in combination with chemotherapeutic agents, which typically block cancer cell proliferation. Even though a key role for PD-L1 in promoting tumor growth has previously been identified, the mechanism of the PD-L1-mediated effect on cancer cell proliferation has not been fully elucidated. Therefore, in-depth mechanistic studies are needed to understand the underlying mechanisms by which PD-L1 intrinsically affects cancer cell proliferation. The results of these studies will form the basis for new combination therapies with improved overall clinical outcomes.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a combination of a Livin inhibitor and an inhibitor of the interaction between PD-1 and its ligand PD-L1, or a combination of a Livin inhibitor and an inhibitor of the intracellular downstream signaling pathway of a complex formed by PD-1 and PD-L1 binding, or a combination of a Livin inhibitor and an inhibitor of the intracellular downstream signaling pathway of PD-1 or PD-L1.

In one embodiment, said inhibitor of the interaction between PD-1 and its ligand PD-L1 is an inhibitor that targets PD-1 and/or an inhibitor that targets PD-L1; wherein, further, said inhibitor of the interaction between PD-1 and its ligand PD-L1 is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the interaction between PD-1 and its ligand PD-L1 is an antibody, an oligopeptide, or a small molecule inhibitor; wherein, more preferably, said inhibitor of the interaction between PD-1 and its ligand PD-L1 is an antibody.

In one embodiment, said inhibitor of the intracellular downstream signaling pathway of a complex formed by PD-1 and PD-L1 binding is an inhibitor that targets PD-1 and/or an inhibitor that targets PD-L1 and/or an inhibitor that targets at least one member of the intracellular downstream signaling pathway of a complex formed by PD-1 and PD-L1 binding; wherein, further, said inhibitor of the intracellular downstream signaling pathway of a complex formed by PD-1 and PD-L1 binding is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the intracellular downstream signaling pathway of a complex formed by PD-1 and PD-L1 binding is an antibody, an oligopeptide, or a small molecule inhibitor; wherein, more preferably, said inhibitor of the intracellular downstream signaling pathway of a complex formed by PD-1 and PD-L1 binding is an antibody.

In one embodiment, said inhibitor of the intracellular downstream signaling pathway of PD-1 or PD-L1 is an inhibitor that targets PD-1 or an inhibitor that targets PD-L1 and/or an inhibitor that targets at least one member of the intracellular downstream signaling pathway of PD-1 or PD-L1; wherein, further, said inhibitor of the intracellular downstream signaling pathway of PD-1 or PD-L1 is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the intracellular downstream signaling pathway of PD-1 or PD-L1 is an antibody, an oligopeptide, or a small molecule inhibitor; wherein, more preferably, said inhibitor of the intracellular downstream signaling pathway of PD-1 or PD-L1 is an antibody.

In one embodiment, said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is selected from the group comprising Pembrolizumab, Nivolumab, Cemiplimab, Dostarlimab (TSR-042), Retifanlimab, Camrelizumab (SHR1210), Sintilimab (IBI308), Toripalimab (JS 001), Tislelizumab (BGB-A317), Spartalizumab (PDR001), AMP-224, AMP-514 (MEDI0680), Acrixolimab (YBL-006), REGN2810, Prolgolimab (BCD-100), PF-0681591, Ezabenlimab (BI-754091), Geptanolimab (GB226), Zimberelimab (GLS-010), LZM009, Pucotenlimab (HX008), Atezolizumab, Avelumab, Durvalumab (Medi4736), Cosibelimab, Envafolimab (KN035), CA-170, AUNP12, BMS-986189, M7824, Pacmilimab (CX-072), FAZ-053, LY-3300054, SHR-1316, and Socazolimab (ZKAB001).

In one embodiment, said Livin inhibitor is selected from a group comprising oligopeptides, small molecule inhibitors, antibodies including bispecific antibodies and nanobodies, vaccines, oligonucleotides, such as aptamers, siRNAs, shRNAs, and nucleic acids such as DNA and RNA, encoding said oligopeptides, antibodies including bispecific antibodies and nanobodies, vaccines, and oligonucleotides, such as aptamers, siRNAs, and shRNAs;
wherein, preferably, said Livin inhibitor is selected from a group comprising oligopeptides, small molecule inhibitors, antibodies, and vaccines;
wherein, more preferably, said Livin inhibitor is an oligopeptide or a small molecule inhibitor.

In one embodiment, said Livin inhibitor is selected from lAP-inhibitors, wherein, preferably, said IAP-inhibitors are selected from a group comprising JQ1, SMAC mimetics such as AZD5582, SM-164, Birinapant (TL32711), GDC-0152, LCL161, and BV6, and a vaccine targeting Livin.

In one embodiment, said Livin inhibitor is selected from a group comprising JQ1, BDB oligopeptides, Livin-binding peptides, and vaccines targeting Livin.

In one embodiment, said combination comprises one or more further agent(s) or drug(s), such as chemotherapeutic drugs.

In a preferred embodiment, said chemotherapeutic drugs are selected from a group comprising Paclitaxel, Docetaxel, Epiribicin, Eribulin, Doxorubicin, Cyclophosphamide, Methotrexate, Fluorouracil, Capecitabine, Carboplatin, Gemcitabine, Nab-paclitaxel, and Vinorelbine; wherein, preferably, said chemotherapeutic drug is Paclitaxel.

In another aspect, the present invention relates to a combination as defined in the foregoing embodiments, for use in a method of treatment of cancer; wherein, preferably, said cancer is selected from breast cancer, hepatocellular carcinoma, melanoma, oesophageal cancer, renal cancer, colon cancer, colorectal cancer, lung cancer, mesothelioma, bladder cancer, gastrointestinal cancer, head and neck squamous cell cancer including nasopharyngeal carcinoma, Hodgkin's lymphoma, gastric/gastroesophageal junction (GEJ) adenocarcinoma, endometrial carcinoma, primary mediastinal large B-cell lymphoma, Merkel cell carcinoma, urothelial cancer, ovarian cancer, and prostate cancer;
wherein, more preferably, said cancer is breast cancer.

In one embodiment, said combination is administered to a patient in need thereof; wherein, preferably, said patient is a mammal; wherein, more preferably, said patient is a human.

In one embodiment, said breast cancer is selected from: triple-negative breast cancer, locally advanced non-inflammatory breast cancer, invasive ductal carcinoma, inflammatory breast cancer, metastatic breast cancer, medullary carcinoma, tubular carcinoma, mucinous carcinoma, adenoid cystic carcinoma of the breast, metaplastic breast cancer, basal type breast cancer, and papillary breast cancer;
optionally, wherein the cells of said breast cancer contain mutations in the BRCA1 and/or BRCA2 gene.

In one embodiment, cells of said breast cancer are characterized by being estrogen receptor-negative, or progesterone receptor-negative, or human epidermal growth factor receptor 2-negative, or any possible combination thereof; wherein, preferably, cells of the cancer are characterized by a combination thereof; wherein, even more preferably, cells of the cancer are characterized by being estrogen receptor-negative, progesterone receptor-negative, and human epidermal growth factor receptor 2-negative, also called triple-negative breast cancer.

In one embodiment, in said method, administering said combination to said patient in need thereof comprises the steps:
(a) administering a dose of said Livin inhibitor to said patient, and
(b) administering a dose of said inhibitor of the interaction between PD-1 and its ligand PD-L1 to said patient; or administering a dose of said inhibitor of the intracellular downstream signaling pathway of a complex formed by PD-1 and PD-L1 binding to said patient; or administering a dose of said inhibitor of the intracellular downstream signaling pathway of PD-1 or PD-L1 to said patient;

wherein step (a) is performed before step (b), or step (b) is performed before step (a), or step (a) and step (b) are performed simultaneously;
further wherein steps (a) and (b) are performed once or wherein steps (a) and (b) are repeated in periodic cycles.

In one embodiment, said combination is administered to a patient in need thereof for a treatment duration ranging from 1 day to lifetime; preferably 1 day to 30 years; more preferably 1 day to 10 years; even more preferably 1 day to 5 years; even more preferably 1 day to 2 years; even more preferably 1 day to 12 months; most preferably 1 day to 6 months.

In one embodiment, in said method of treatment, said combination for use is administered in a pharmaceutical composition, said pharmaceutical composition comprising pharmaceutically acceptable excipient(s) and/or carrier(s).

In one embodiment, in said method of treatment, said combination is supplemented to an ongoing therapy for the treatment of breast cancer.

In one embodiment, said Livin inhibitor is formulated and administered as an injection intravenously or subcutaneously, a pill, a tablet, a capsule, a suppository, an oral solution, a buccal, a granule, a powder, a suspension, a solution, a spray, an ointment, drops, or patches; preferably an injection intravenously or subcutaneously;
wherein, further, said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is formulated and administered as an injection intravenously or subcutaneously, a pill, a tablet, a capsule, a suppository, an oral solution, a buccal, a granule, a powder, a suspension, a solution, a spray, an ointment, drops, or patches; preferably an injection intravenously or subcutaneously.

In another aspect, the present invention relates to a kit for the preparation of an infusion solution for the administration of a combination of a Livin inhibitor and an inhibitor of the interaction between PD-1 and its ligand PD-L1, or a kit for the preparation of an infusion solution for the administration of a combination of a Livin inhibitor and an inhibitor of the intracellular downstream signaling pathway of a complex formed by PD-1 and PD-L1 binding, or a kit for the preparation of an infusion solution for the administration of a combination of a Livin inhibitor and an inhibitor of the intracellular downstream signaling pathway of PD-1 or PD-L1, as defined in any of the foregoing embodiments, said kit comprising either: (A) a first container containing a composition comprising a Livin inhibitor, and a second container containing a composition comprising a pharmaceutically acceptable diluent; and
a third container containing a composition comprising an inhibitor of the interaction between PD-1 and its ligand PD-L1, or an inhibitor of the intracellular downstream signaling pathway of a complex formed by PD-1 and PD-L1 binding, or an inhibitor of the intracellular downstream signaling pathway of PD-1 or PD-L1, and a fourth container containing a composition comprising a pharmaceutically acceptable diluent;
   or said kit comprising:
   (B) a first container containing said Livin inhibitor pre-mixed with said inhibitor of the interaction between PD-1 and its ligand PD-L1; or a first container containing said Livin inhibitor pre-mixed with said inhibitor of the intracellular downstream signaling pathway of a complex formed by PD-1 and PD-L1 binding; or a first container containing said Livin inhibitor pre-mixed with said inhibitor of the intracellular downstream signaling pathway of PD-1 or PD-L1; and a second container containing a composition comprising a pharmaceutically acceptable diluent;
wherein said containers are preferably selected from vials, bottles, pre-filled syringes, ampoules, and infusion bags.

In one embodiment, in said kit, said Livin inhibitor is provided in a form selected from: an injection concentrate, a pre-diluted solution, and a lyophilized powder for reconstitution;
wherein said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is provided in a form selected from: an injection concentrate, a pre-diluted solution, and a lyophilized powder for reconstitution.

Another aspect of the present application relates to a method of treating a patient, wherein said method comprises administering a combination of a Livin inhibitor and an inhibitor of the interaction between PD-1 and its ligand PD-L1, or administering a combination of a Livin inhibitor and an inhibitor of the intracellular downstream signaling pathway of a complex formed by PD-1 and PD-L1 binding, or administering a combination of a Livin inhibitor and an inhibitor of the intracellular downstream signaling pathway of PD-1 or PD-L1, to a patient in need thereof; preferably wherein said patient is a mammal; more preferably wherein said patient is a human.

According to this aspect, the cancer, the patient, the combination, and the method of treatment are as defined herein.

Another aspect of the present application is a use of a combination of a Livin inhibitor and an inhibitor of the interaction between PD-1 and its ligand PD-L1, or a combination of a Livin inhibitor and an inhibitor of the intracellular downstream signaling pathway of a complex formed by PD-1 and PD-L1 binding, or a combination of a Livin inhibitor and an inhibitor of the intracellular downstream signaling pathway of PD-1 or PD-L1, for the manufacture of a medicament for treatment of a cancer in a patient, preferably wherein said patient is a mammal; more preferably wherein said patient is a human.

According to this aspect, the cancer, the patient, the combination, and the method of treatment are as defined herein.

### DETAILED DESCRIPTION

In one embodiment, the term "patient" relates to a human or an animal, preferably a human.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "Livin", as used herein, refers to a protein encoded by the *BIRC7* gene. Livin (also known as ML-IAP and RNF50) is a member of the family of inhibitor of apoptosis proteins (IAP) and contains a single copy of a baculovirus IAP repeat (BIR) as well as a RING-type zinc finger domain.

The term "inhibitor", as used herein, refers to a substance or a molecule that interacts with a protein or its interaction partner(s) to slow down, reduce or prevent the activity of the protein.

The term "PD-1", as used herein, refers to Programmed cell death protein 1 (PD-1), also known as CD279 (cluster of differentiation 279). PD-1 is a protein on the surface of T and B cells that has a role in regulating the immune system's response to the cells of the human body by downregulating the immune system and promoting self-tolerance by suppressing T cell inflammatory activity. PD-1 is a type I membrane protein of 288 amino acids and is an immune checkpoint. In humans it is encoded by the *PDCD1* gene.

The term "PD-L1", as used herein, refers to Programmed death-ligand 1 (PD-L1), a 40kDa type 1 transmembrane protein that is encoded by the human *CD274* gene. PD-L1 is a ligand of PD-1. PD-L1 is also known as cluster of differentiation 274 (CD274) or B7 homolog 1.

The term "downstream signaling pathway", as used herein, refers to a cascade of signal activation or inhibition (including enzyme, protein conformational change, ion channel opening or closure) that drive a cell to respond to signals of a triggering event. In this regard, the "intracellular downstream signaling pathway of PD-1", as used herein, refers to a cascade of intracellular signal activation or inhibition that drive a cell to respond to the activity of PD-1 alone. The term "intracellular downstream signaling pathway of PD-L1", as used herein, refers to a cascade of intracellular signal activation or inhibition that drive a cell to respond to the activity of PD-L1 alone. The term "intracellular downstream signaling pathway of a complex formed by PD-1 and PD-L1 binding", as used herein, refers to a cascade of intracellular enzyme activation or inhibition that drive a cell to respond to the formation of a complex of PD-1 and PD-L1, formed upon binding of PD-1 and its ligand PD-L1.

The term "member of the intracellular downstream signaling pathway", as used herein, refers to a molecule, such as a protein, that is involved in the intracellular signaling pathway. Members of the intracellular downstream signaling pathway may be signaling molecules, ion channels, ligands, or enzymes.

The term "bispecific antibody", as used herein, refers to a type of antibody that can bind to two different antigens at the same time.

The term "nanobody", as used herein, refers to a single-domain antibody (sdAb), which is an antibody fragment consisting of a single monomeric variable antibody domain. Like a whole antibody, it is able to bind selectively to a specific antigen.

The term "fusion proteins", as used herein, refers to chimeric proteins that are created through the joining of two or more genes that originally coded for separate proteins.

The term "antibody prodrugs", as used herein, refers to masked antibodies engineered to be pharmacologically inactive until they are activated by proteases in the diseased tissue microenvironment.

The term "aptamers", as used herein, refers to short sequences of artificial DNA, RNA, XNA, or peptide that bind a specific target molecule, or family of target molecules. They exhibit a range of affinities, with variable levels of off-target binding.

The term "vaccine", as used herein, refers to a preparation that is used to stimulate the body's immune response against a specific target.

The term "IAP", as used herein, refers to Inhibitors of apoptosis proteins (IAP), which are a family of functionally and structurally related proteins that serve as negative regulators of both caspases and cell death. If mutated or improperly regulated, they can frequently lead to cancer or other effects.

The term "SMAC mimetics", as used herein, refers to mimetics of second mitochondria-derived activator of caspases (SMAC).

The term "BDB oligopeptides", as used herein, refers to oligopeptides that specifically bind Livin and release the inhibitory effect that Livin has on caspase activity. BDB oligopeptides are specific inhibitors of Livin.

The term "Livin binding peptides", as used herein, refers to a group of peptides that specifically bind Livin and inhibit its negative regulatory effect on caspases and cell death.

The term "triple-negative breast cancer" (TNBC), as used herein, refers to a type of breast cancer wherein the cells are characterized as by being estrogen receptor negative, progesterone receptor negative, and human epidermal growth factor receptor 2 negative.

The term "estrogen receptor negative" (ER negative), as used herein, refers to the respective hormone receptor status. The hormone receptor status was considered negative if <1% of the tumor cells were stained during immunohistochemistry when using a stain targeting the estrogen receptor.

The term "progesterone receptor negative" (PR negative), as used herein, refers to the respective hormone receptor status. The hormone receptor status was considered negative if <1% of the tumor cells were stained during immunohistochemistry when using a stain targeting the progesterone receptor.

The term "human epidermal growth factor receptor 2 negative" (HER2 negative), as used herein, refers to the respective Her2 status. The Her2 status was considered negative if <10% of the tumor cells were stained during immunohistochemistry (+3 intensity or +2 with a situ hybridization (ISH) positive test), when using a stain targeting the human epidermal growth factor receptor 2.

The term "combination", as used herein, refers to the result of an act of combining entities, materials, compounds, or compositions in a spatial and/or temporal manner. In such combination, the combined entities, materials, compounds, or compositions can be administered together in a single composition or separately as separate entities, albeit at the same time, or one immediately after the other. Alternatively, in such combinations, the combined entities, materials, compounds, or compositions can be administered in a temporally separate manner, with intervals between the administration of the separate individual entities, materials, compounds, or compositions. A combination can also mean the joint use of the active ingredients in a method of treatment to achieve a beneficial effect, especially a synergistic therapeutic effect. For this joint use, the active ingredients can be administered together at the same time or in the form of mixed composition. Alternatively, a combination can also mean a separate, temporally distinct administration of the respective active ingredient in a defined dosing regimen, such defined dosing regimen comprising, however, the separate administration of all the ingredients that form part of the combination.

The term "administering", as used herein, refers to the application of a compound to a patient (either human or animal) having or suspected of having cancer. Said application can be performed as an injection intravenously or subcutaneously.

The term "simultaneous administration" and the like, as used herein, refers to the joint administration of active ingredients at the same time.

The term "invasive ductal carcinoma", as used herein, refers to an invasive cancer where abnormal cancer cells that began forming in the milk ducts have spread beyond the ducts into other parts of the breast tissue. Invasive cancer cells can also spread to other parts of the body. It is also sometimes called infiltrative ductal carcinoma.

The term "inflammatory breast cancer", as used herein, refers to an aggressive and fastgrowing breast cancer in which cancer cells infiltrate the skin and lymph vessels of the breast.

It often produces no distinct tumor or lump that can be felt and isolated within the breast. But when the lymph vessels become blocked by the breast cancer cells, symptoms begin to appear.

The term "metastatic breast cancer", as used herein, refers to a cancer that has spread to other parts of the body. This usually includes the lungs, liver, bones and/or brain.

The term "medullary carcinoma", as used herein, refers to an invasive carcinoma, as a part of breast cancer.

The term "tubular carcinoma", as used herein, refers to an invasive carcinoma, as a part of breast cancer.

The term "mucinous carcinoma", as used herein, refers to an invasive carcinoma which starts in the main cells of mucus, called mucin.

The term "adenoid cystic carcinoma of the breast", as used herein, refers to an invasive carcinoma, as an aggressive type of breast cancer.

The term "metaplastic breast cancer", as used herein, refers to an invasive carcinoma, as an aggressive type of breast cancer.

The term "basal type breast cancer", as used herein, refers to a unique type of breast cancer with particular genetic changes in the cells. The cells produce large amounts of cytokeratin 5/6 and usually are TNBC.

The term "papillary breast cancer", as used herein, refers to an invasive carcinoma with benign behavior.

The term "locally advanced non-inflammatory breast cancer", as used herein, refers to breast cancer including those with stage IIIA-C breast tumors with metastases to ipsilateral axillary or internal mammary lymph nodes, or with direct extension into chest wall or through skin, without distant metastases.

The terms "BRCA1" and "BRCA2", as used herein, refer to the human tumor suppressor genes "breast cancer gene 1" and "breast cancer gene 2", respectively. People with inherited harmful mutations in one of these genes have increased risks of several cancers-most notably breast and ovarian cancer, but also several additional types of cancer.

The term "kit" as used herein, refers to a package, a box, a carton, a bag, or a blister, containing one or more different compounds and/or their respective diluents necessary for the administration of said compounds.

The term "pharmaceutically acceptable diluent", as used herein, refers to a solution suitable for dilution or reconstitution of the respective active ingredients. Pharmaceutically acceptable diluents are well tolerated when applied to a patient and may be used to adjust the concentration of active ingredients to the desired range for application. When provided in a kit, the composition of pharmaceutically acceptable diluents may be different for each active ingredient that is to be diluted. The composition of the diluents depends, for example, on the solubility of the respective active ingredient in different solvents.

### BRIEF DESCRIPTION OF THE FIGURES

The present invention is now further described by reference to the following figures.

All methods mentioned in the figure descriptions below were carried out as described in detail in the examples.

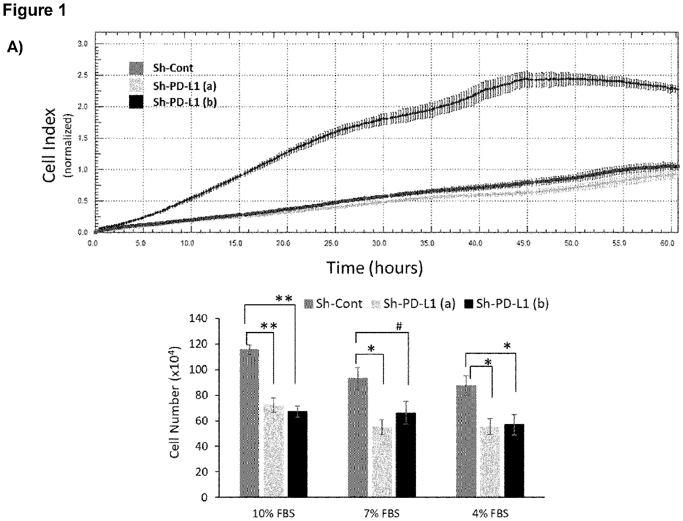

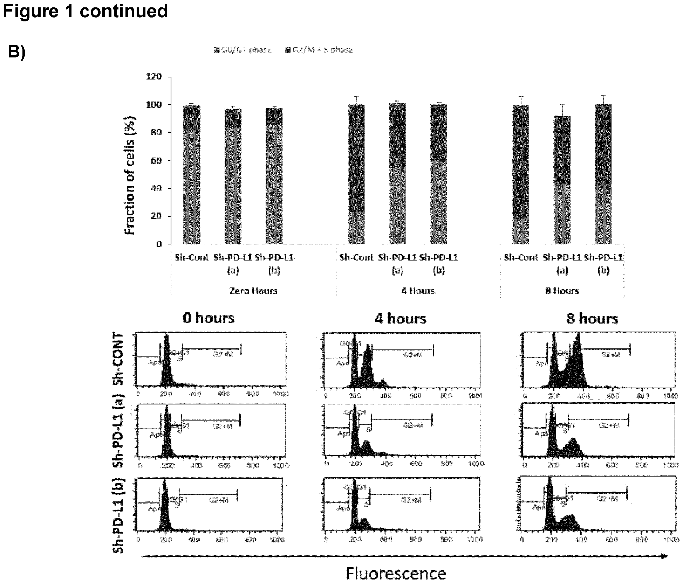

### Figure 1: PD-L1 enhances the proliferation of MDA-MB-231 cells.

A) Cell proliferation analysis of PD-L1^{KD} MDA-MB-231 cells (Sh-PD-L1(a), ShPD-L1(b)) and their control PD-L1^{Pos} (Sh-Cont) using (top) RTCA system (n=1, cells were cultured in complete 10% FBS) or (bottom) manual counting (n=3, cells were cultured in 4-10% FBS).
B) Cell cycle analysis using propidium iodide-stained cell flow cytometry after drug (aphidicolin) washing and complete medium supplementation. Top) Data are presented as the percentage of cells in either G0/G1 or S-G2/M (i.e., cycling) phases and displayed as the mean of 3 independent experiments ± SEM. Bottom) Representative flow cytometry cell cycle analysis.

### Figure 2: PD-L1 expression enhances the proliferation of SUM159 cells.

Cell proliferation (mean ± SEM) of PD-L1^{KD} SUM159 clones (KD1 and KD2) and their control (Sh-C) using (top) RTCA system (n=1) or (bottom) manual counting.

### Figure 3: Downregulation of EIF1AX in PD-L1^{Pos} cells confirmed by Western Blotting.

Expression of EIF1AX in the PD-L1^{KD} clones Sh-PD-L1(a) and Sh-PD-L1(b) of MDA-MB-231 cells compared with the control (Sh-Cont) measured by western blot (left) with quantification (mean ± SEM) of blots from 3 different experiments (right).

### Figure 4: Proteomic analysis of nuclear extracts from PD-L1^{Pos} cells show cell cycle and mitosis-related proteins.

Protein expression levels (normalized) of the top 25 proteins upregulated in PD-L1^{Pos} control cells (A). Gene/protein enrichment analysis of nuclear extracts using ShinyGo Gene Ontology (GO) cellular component analysis (B), GO Biological process analysis of differentially upregulated proteins in PD-L1^{Pos} (C) or PD-L1^{KD} cells (D).

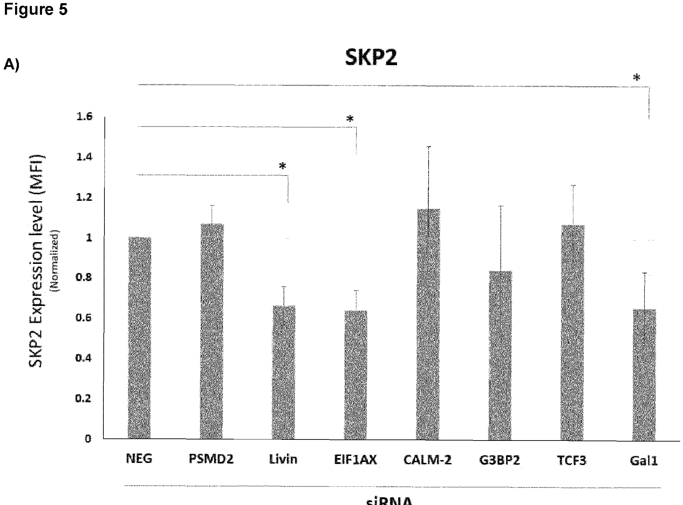

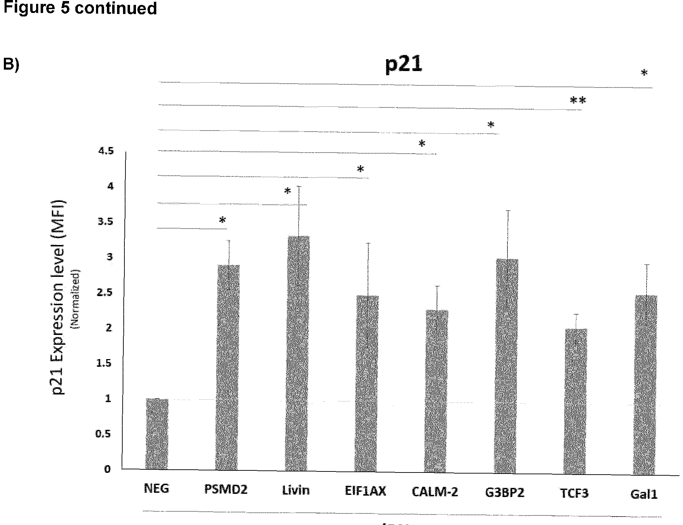

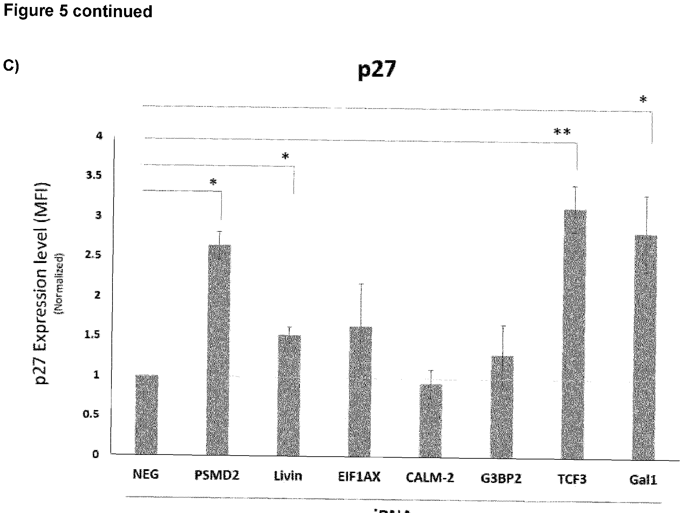

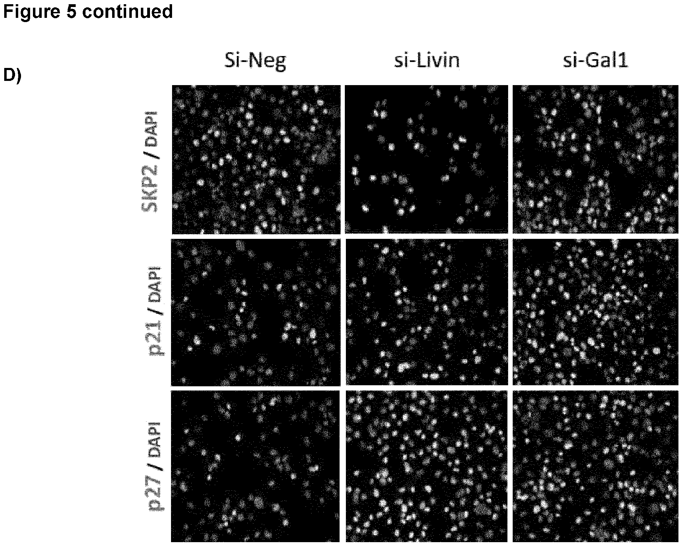

### Figure 5: KD of Livin and Gal1 modulates SKP2/p21/p27 expression.

The expression levels of SKP2 (A), p21 (B), and p27 (C) in **MDA-MB-231** cells upon transient gene expression knockdown (KD) of possible upstream proteins as measured by qIF. Data were normalized to the negative control siRNA (NEG siRNA) and are displayed as the mean of 4 independent experiments ± SEM. D) Representative IF images for SKP2, p21, and p27 expression upon KD of Livin or Gal1 compared with the control siRNA. Data were normalized to the colonies formed by the NEG siRNA-treated cells and are displayed as the mean of at least 3 independent experiments ± SEM.

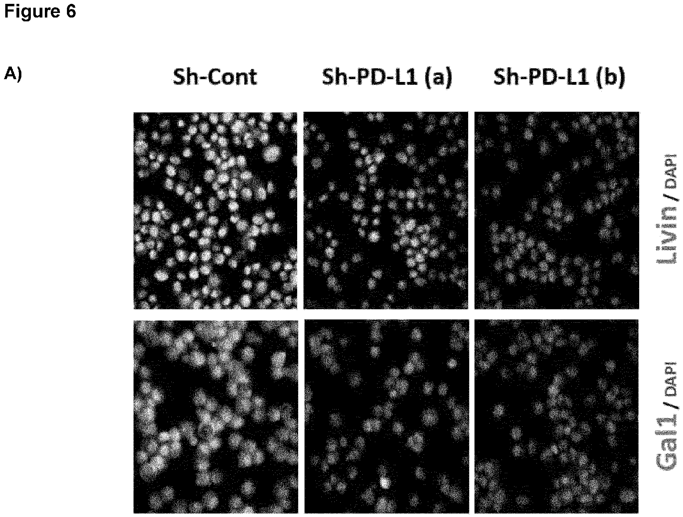

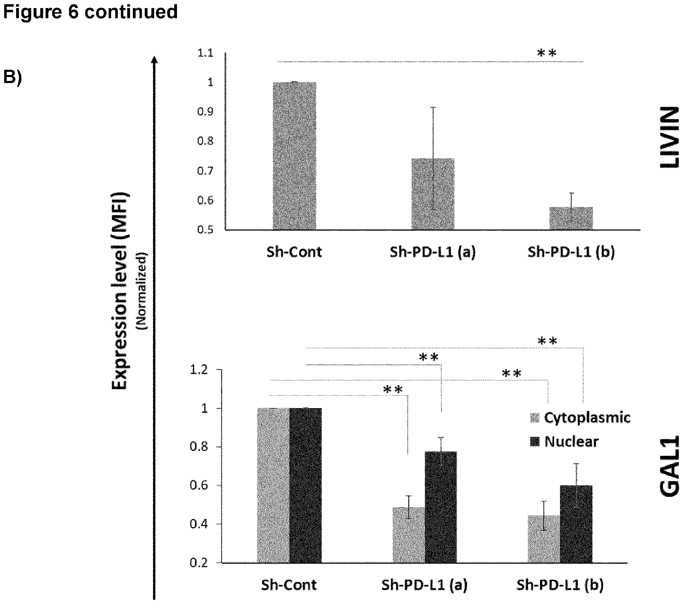

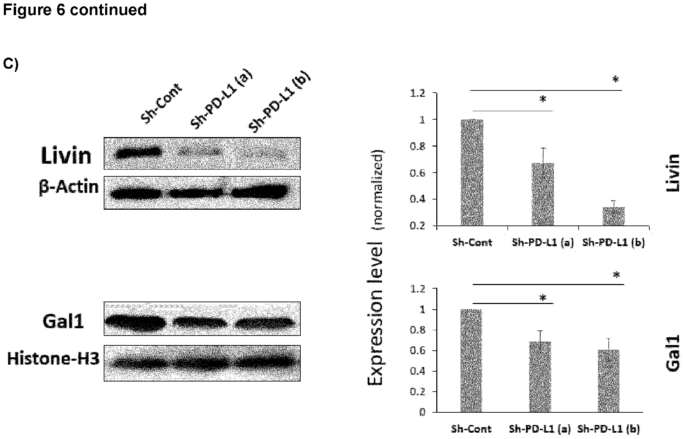

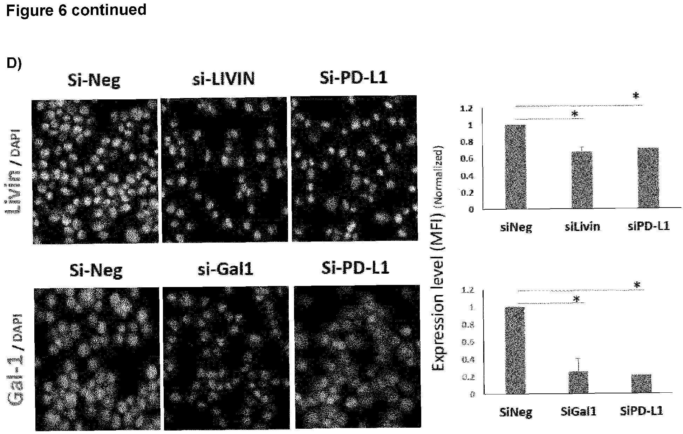

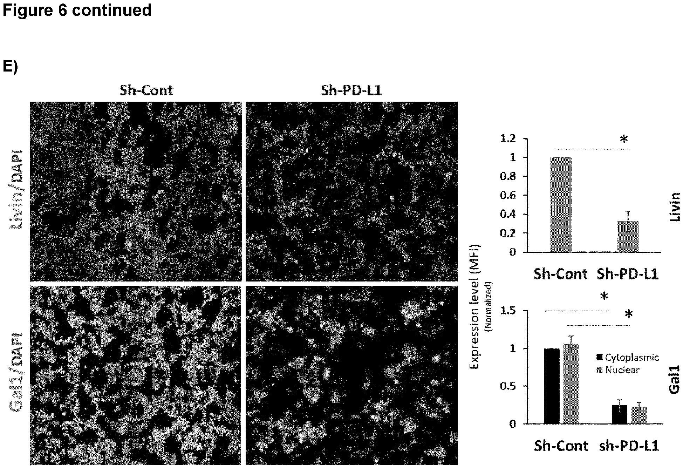

### Figure 6: PD-L1 modulates the expression of Livin and Gal1 in BC cells

A) Representative IF images of the ShRNA PD-L1^{KD} clones and the control (at ×200 magnification). B) The expression level of Livin and Gal1 in the ShRNA PD-L1^{KD} clones of MDA-MB-231 cells compared with the PD-L1^{Pos} control as measured by qIF. Data were normalized on the control (Sh-Cont) MFI and displayed as mean of 4 independent experiments ± SEM. C) Western blots showing Livin and Gal1 expression following ShRNA PD-L1 KD in MDA-MB-231 cells (left) with quantification (mean ± SEM) of blots from 3 different experiments (right). D) Right) qIF showing MFI of Livin and Gal1 in MDA-MB-231 upon transient KD of PD-L1 Livin or Gal1 (using SiRNA) after normalization on the control (Si-Neg) displayed as a mean ± SEM of 4 independent experiments. Left) Representative IF images upon transient KD or siNeg (at ×200 magnification). E) Expression of Livin and Gal1 in PD-L1^{Pos} and ShRNA PD-L1^{KD} MDA-MB-231 xenografts. Representative IF images (at ×100 magnification) (left) and a bar graph showing the expression level (mean ± SEM) from 3 different experiments as measured by qIF after normalization to the control xenograft cells (Sh-Cont).

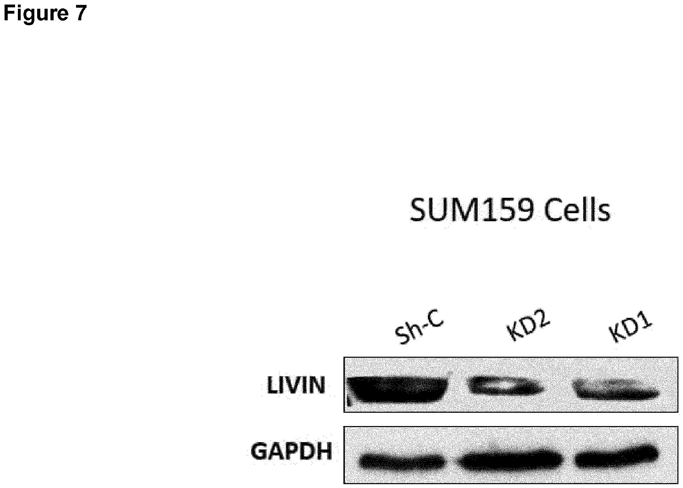

### Figure 7: PD-L1 expression modulates the expression of Livin in SUM159 breast cancer cells.

Western Blot showing the expression of Livin in SUM159 PD-L1^{KD} clones (KD1 and KD2) as compared to the control (Sh-C).

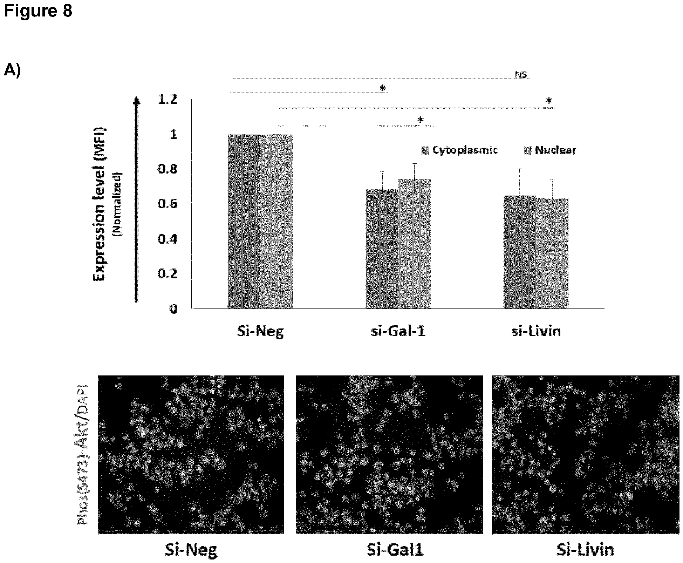

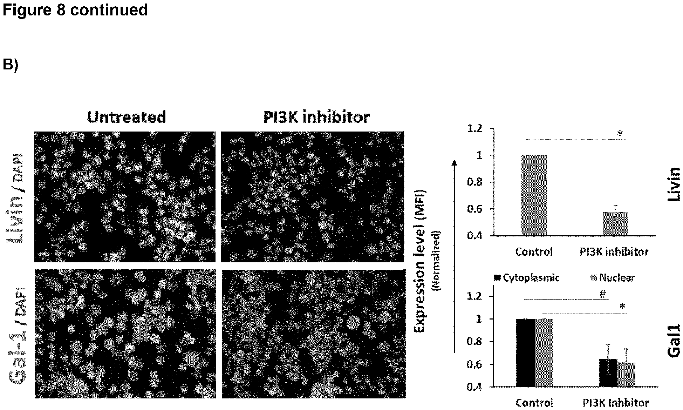

### Figure 8: Livin/Gal1 expression activates PI3K/AKT pathway in a positive feedback loop.

A, Top) qIF showing MFI of phospho (S473) p-AKT upon transient KD of Livin or Gal1 in MDA-MB-231 cells as compared with the control siRNA (siNeg) after normalization that is displayed as mean ± SEM of 3 independent experiments. Bottom) Representative IF images (at ×200 magnification). B, Left) Representative IF images (at ×200 magnification) showing Gal1 and Livin expression upon treatment with LY294002. Right) Bar graph showing mean ± SEM of normalized data from 3 independent experiments.

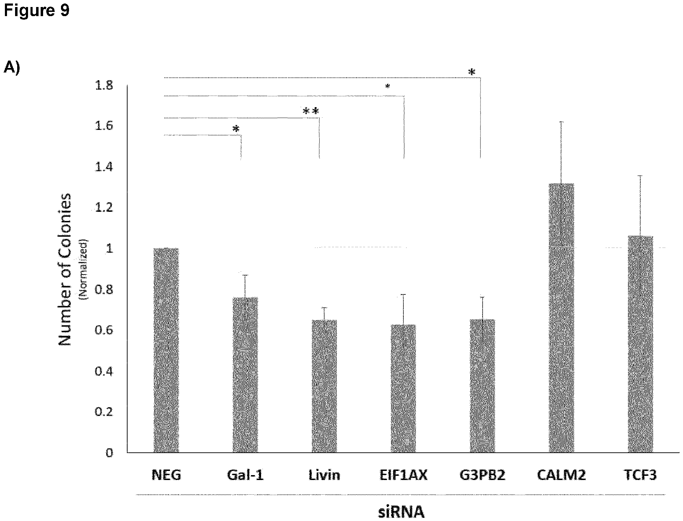

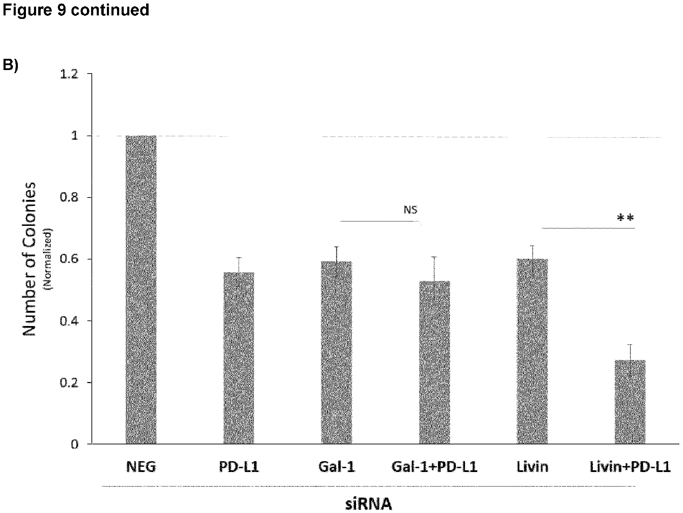

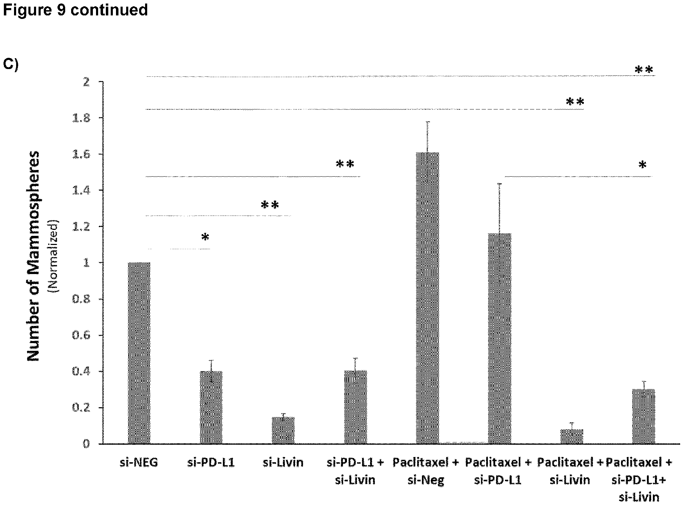

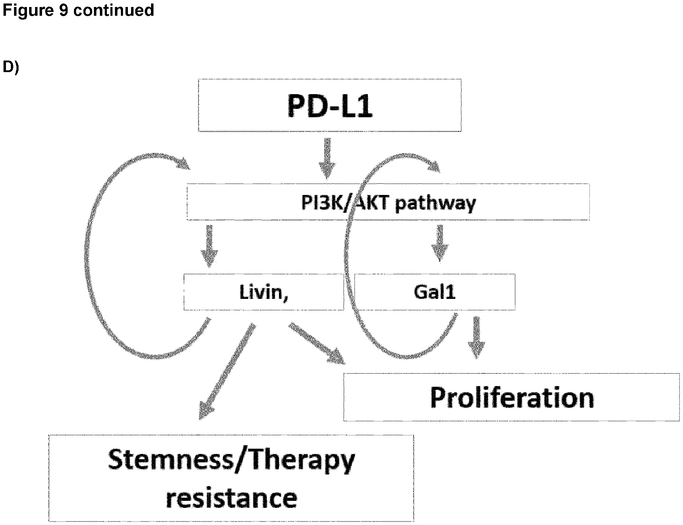

### Figure 9: Livin and PD-L1 promotes colony formation and supports sternness of breast cancer cells.

The sternness of cancer cells were assessed the colony-forming assay (CFA) or mammosphere forming assay of MDA-MB-231 cancer cells. A&B) CFA after transient KD of several PD-L1 downstream proteins alone (A) or in combination with PD-L1 (B). C) Mammosphere formation upon transient KD of Livin alone or in combination with PD-L1 KD, in the presence of absence of the chemotherapeutic agent, Paclitaxel. D) Schematic diagram depicting the effect of PD-L1 on promoting PI3K/AKT activation through Livin and Gal1, which sustain PI3K/AKT signaling through positive feedback loops. Consequently, this process unleashes cell cycle progression and cancer cell sternness. Inhibition of Livin and PD-L1 inhibits proliferation, sternness and resistance to chemotherapy. *, ** indicates statistical significance, *=p<0.05, ** p<0.001.

In the following, reference is made to the examples, which are given to illustrate, not to limit the present invention.

### EXAMPLES

The following examples demonstrate for the first time the effect of the combination of Livin inhibition and PD-L1 inhibition. Exemplified by the breast cancer cell lines MDA-MB-231 and SUM159PT, the inventors elucidate the intracellular connection between PD-1/PD-L1 and Livin activity and describe the anti-proliferative effect of their combined knockdowns. Without wishing to be bound by any theory, the inventors therefore surmise that the anti-proliferative effect of this combination, as exemplified in the breast cancer cell lines, can be extrapolated to other types of cancer.

### Materials and Methods

### Cell Culture and treatment

The MDA-MB-231 (CVCL_0062) cell line (ATCC, USA) was maintained in high glucose DMEM medium supplemented with 4-10% fetal bovine serum (FBS) (Sigma, St. Louis, USA), while SUM159PT (CVCL_5423) cells (Asterand, USA) were cultured and maintained in DMEM/F12 medium supplemented with 5% FBS, 1µg/mL hydrocortisone, and 5 µg/mL insulin (both from Sigma, USA). All cell lines used are listed using the official cell line name and its Research Resource Identifier (RRID) as available in the ExPASy Cellosaurus database. In addition, the source/suppliers of all cell lines used has/have been provided. All experiments were performed with mycoplasma-free cells.

Protein expression was measured in the exponential phase (24-48h) of cell culture. To minimize the effect of the well-established feedback loops in the PI3K/AKT pathway, cells were serum-starved for 24-30h, and media were exchanged with complete (serum-supplemented) medium in an 18-24h culture before analyzing the protein expression of Livin, and Gal1.

### Gene expression knockdown

The PD-L1 knockdown (PD-L1^{KD}) stable clones, sh-PD-L1(a) and sh-PD-L1(b), and their scrambled shRNA control (Sh-Cont) were generated by transfection of MDA-MB-231 cells with specific shRNA against PD-L1 from OriGene, USA (RS vector, TR314098) using Lipofectamine LTX (Thermo Fisher Scientific, USA). Similarly, PD-L1^{KD} clones of SUM159PT cells were generated using the same shRNA vector (TR314098) and designated as KD1 and KD2 and their scrambled shRNA control is Sh-C.

For transient gene KD, cells were transfected with siRNA according to the manufacturer's instructions using Lipofectamine RNAiMAX transfection reagent (Thermo Fisher Scientific, USA).

In-house siRNAs were designed using the Kay Lab siRNA/shRNA/Oligo Optimal Design website (http://web.stanford.edu/group/markkaylab/cgi-bin/) and were synthesized by Metabion International AG (Germany) (Table 1).

**Table 1. SiRNA sequence**

| **Target** | **Sequence** | |
|---|---|---|
| | **Sense** | **Antisense** |
| PD-L1 | GGC AUU UGC UGA ACG CAU Utt (SEQ ID No.1) | AAU GCG UUC AGC AAA UGC Cag (SEQ ID No.2) |
| PSMD2 | UAU ACA UAG UGU GAU UUG Ctt (SEQ ID No.3) | GCA AAU CAC ACU AUG UAU Att (SEQ ID No.4) |
| Livin | GGA AGA GAC UUU GUC CAC Att (SEQ ID No.5) | UGU GGA CAA AGU CUC UUC Ctt (SEQ ID No. 6) |
| EIF1AX | UUU AUU UAU GAU UAA AUC Ctt (SEQ ID No.7) | GGA UUU AAU CAU AAA UAA Att (SEQ ID No. 8) |
| CALM2 | UAA AUU UAA CAC AUU CUG Ctt (SEQ ID No.9) | GCA GAA UGU GUU AAA UUU Att (SEQ ID No.10) |
| G3BP2 | UAU AUA UAU AUA UAU AUG Ctt (SEQ ID No. 11) | GCA UAU AUA UAU AUA UAU Att (SEQ ID No. 12) |
| TCF-3 | UUA AUA CAA CGU UUA AUC Ctt (SEQ ID No. 13) | GGA UUA AAC GUU GUA UUA Att (SEQ ID No. 14) |
| Gal-1 | UUU GAA UUC GUA UCC AUC Ctt (SEQ ID No. 15) | GGA UGG AUA CGA AUU CAA Att (SEQ ID No. 16) |
| SKP-2 | UUA UAU AUG GAU AGU UUC Ctt (SEQ ID No. 17) | GGA AAC UAU CCA UAU AUA Att (SEQ ID No. 18) |

### Proteomic analysis:

Cytoplasmic and nuclear proteins were fractionated and were validated using GAPDH and histone H3, respectively, using NP-40, which lyse cells, while keeping the nuclear membranes intact.

Isolated nuclear proteins were further subjected to in-solution tryptic digestion. Individual proteins were identified by one-dimensional Nano Acquity liquid chromatography coupled with tandem mass spectrometry on Synapt G2 HDMS (Waters, Manchester, UK). The data were filtered to show only unambiguous protein identification using multiple parameters, including the expected molecular mass, percentage coverage, peptide count, unique peptides, and confidence scores.

### Cell cycle analysis

Cells were seeded overnight, washed with PBS, and serum-starved for 24h before treatment with aphidicolin (4µg/mL) overnight. Cells were washed twice with PBS, trypsinized, and seeded in complete media. At the indicated time points, cells were harvested, fixed with 70% ethanol, stained with Propidium Iodide, acquired using a BD FACSCalibur, and analyzed with BD CellQuest^{™} Pro software (both from BD Biosciences).

### Colony-forming assay

The cells were seeded in a 6-well plate in complete DMEM at a density of 500 cells/well for 14 days. Colonies were washed with PBS, fixed in 4% formaldehyde, stained with crystal violet and counted manually.

### Xenograft and tissue processing

Xenograft tumors in nude mice generated from orthotopic injection of PD-L1^{KD} MDA-MB-231 cells (Sh-PD-L1(a)) or their control (Sh-Cont), were cut into small pieces, digested in a collagenase digestion medium (Stem Cell Technologies, Vancouver, Canada), and agitated at 37°C for 1h. Single cells were cryopreserved in 10% DMSO-based freezing medium.

### Cell proliferation assays

### Manual

Cells (3.5×10⁴/T25 flask) were seeded in a complete medium with 4-10% of FBS. After 72h, cells were collected and counted using the trypan blue exclusion dye and a hemocytometer.

### Automated

The xCELLigence Real-Time Cell Analyzer system (RTCA) (ACEA Biosciences) was used to monitor the cell proliferation over time. Cells were seeded at 20,000 cells/well of an E-plate in 200/µL of medium. Changes in the electronic impedance directly correlates with cell number and the rate of change in the cell index (CI) was calculated automatically using RTCA Software Package 2.0. The data was normalized to the signal recorded 10h after seeding to disregard unattached/dead cells due to the transfection procedure.

### Western blotting

Proteins were separated by SDS-PAGE and transferred to the PVDF membrane. Membranes were incubated with primary antibodies (Table 2) diluted in TBST as per the antibody data sheet, followed by the appropriate secondary antibody. The signal was developed using a SuperSignal kit and visualized using an ImageQuant LAS4010 Biomolecular Imager (GE Healthcare, Pittsburgh, PA, USA).

**Table 2. List of Antibodies used**

| **Target** | **Clone** | **Cat#** | **Company, country** |
|---|---|---|---|
| **Livin** | **D61D1** | **5471** | **CST** |
| **Gal1** | **D608T** | **12936** | **CST** |
| **SKP2** | **D3G5** | **2652** | **CST** |
| **p27** | **D69C12** | **3686** | **CST** |
| **p21** | **12D1** | **2947** | **CST** |
| **EIF1AX** | **poly** | **PA007506DSR2HU** | **Cusabio Technology, China** |
| **Cyclin D1** | **Inhouse** | **N/A** | **Abeam, UK** |
| **FOXO3a** | **D19A7** | **12829** | **CST** |
| **HIF1a** | **D5F3M** | **79233** | **CST** |
| **CST= Cell Signaling Technology, Danvers, MA, USA, Gal1 = Galectin-1** | | | |

### Quantitative immunofluorescence (qIF)

Protein expression analysis using immunofluorescence (IF) was performed on exponentially growing cells. Cells were cytospinned, air-dried (3-24h), and immunostained as per cell signaling technology protocol. Primary antibodies are listed in Table 2.

A BD pathway 855 Image analyzer and BD AttoVision image acquisition software (BD Biosciences) were used for image capturing, and fluorescence quantitation, respectively using 20X objective (Olympus, NA 0.75). Data were analyzed and the Mean Fluorescence Intensities (MFI) were calculated according to instrument standard protocols. Briefly, pre-defined analysis protocols (macros) were formed using a montage of 3×3, which had at least 2000 cells per montaged image. Data were analyzed in BD Image Data Explorer and the images from at least 4 different experiments were used to calculate the expression level of each studied protein. In addition, the number of cells considered positive for each studied protein was quantified using an arbitrary MFI cutoff that selects for around 50% of total analyzed cells for each experiment. All data were further normalized on the untreated control (Sh-Cont).

### Bioinformatics analysis

An in-depth analysis of gene/protein sets obtained from proteomic analysis was performed using ShinyGO, a web-based application (http://bioinformatics.sdstate.edu/go/) for Gene Ontology Enrichment Analysis based on hypergeometric distribution followed by false discovery rate (FDR) correction using a cutoff of 0.1. Gene Ontology (GO)-Biological process analysis was used to link genes with cellular function and GO-Cellular compartment to link genes with different cellular compartments.

### Statistical Analysis

All results were normalized to the untreated cells, and data are represented as the mean ± SEM. Statistical significance between groups was analyzed using paired Student's t-test and displayed as *= p-value < 0.05, **= p-value <0.01, *** = p-value < 0.001, and # indicates borderline significance.

### Ethics approval

This work was reviewed by designated IRB and funded by King Faisal Specialist Hospital and Research Centre project (RAC# 2190-002).

### Example 1: PD-L1 promotes the proliferation of BC cells

The present inventors previously observed larger tumors in PD-L1-expressing (PD-L1^{Pos}) xenografts than their PD-L1 knockdown (PD-L1^{KD}) counterparts. Whether the PD-L1-mediated increase in tumor size was due to extrinsic factors within the microenvironment or an intrinsic effect of PD-L1 expression on BC cells warranted further investigation. The inventors then focused on the effect of PD-L1 on cells proliferation as a fundamental tumorigenic feature of cancer cells that can increase tumor size.

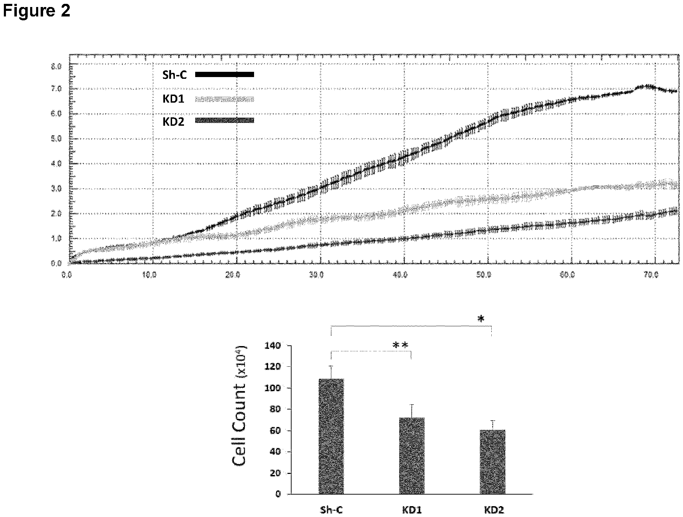

PD-L1^{Pos} MDA-MB-231 BC cells proliferated significantly faster than their PD-L1^{KD} clones PD-L1(a) and PD-L1(b), as demonstrated real-time using RTCA in a three-day culture (Figure 1A, Top). Similarly, a manual assay showed higher (35-40%) proliferation of PD-L1^{Pos} control as compared to PD-L1^{KD} clones (Figure 1A, bottom). Interestingly, the higher proliferation rate of PD-L1^{Pos} BC cells was maintained even at lower serum concentrations (4 and 7%), suggesting intrinsic features that are less dependent on growth-promoting factors in serum-supplemented culture medium (Figure 1A, bottom). The effect of PD-L1 expression on promoting proliferation was confirmed in another BC cell line, SUM159PT (SUM159). PD-L1^{Pos} SUM159 cells (Sh-C) proliferated significantly faster than their knockdown (KD) clones (KD1 and KD2) (Figure 2).

To further examine the effect of PD-L1 on cell proliferation, its effect on the cell cycle of MDA-MB-231 BC cells was studied. Random analysis using flow cytometry did not reveal a major change in cell cycle phases between PD-L1^{KD} cells and their PD-L1^{Pos} counterparts (data not shown). The inventors hypothesized that the rate of cell cycle entry would be higher in PD-L1^{Pos} cells. To this end, cells were synchronized using aphidicolin (4 µg/mL), a reversible inhibitor that blocks the cell cycle progression at the G1/S entry restriction checkpoint. Within hours of drug washout, PD-L1^{Pos} cells progressed into the other phases of the cell cycle (S and G2/M) significantly faster than their PD-L1^{KD} counterparts (Figure 1B). Taken together, these data support the role of PD-L1 in promoting cancer cell proliferation by accelerating cell cycle entry.

### Example 2: Nuclear proteins of PD-L1^{Pos} cells hint for the involvement of proliferation-related key molecules.

The role of PD-L1 expression in the activation of the PI3K/AKT pathway has previously been shown, which is consistent with the increased proliferation. However, PI3K/AKT is a major complex signaling network entangled with many other regulatory pathways in cancer cells. In order to decipher how PD-L1-induced activation of the PI3K/AKT pathway promotes cell proliferation, the present inventors used a bottom-up approach. The inventors hypothesized that downstream regulators would eventually involve nuclear proteins to transcribe genes related to cell proliferation. This is in addition to the possibility of nuclear nPD-L1 interacting directly with proliferation-related proteins in the nucleus.

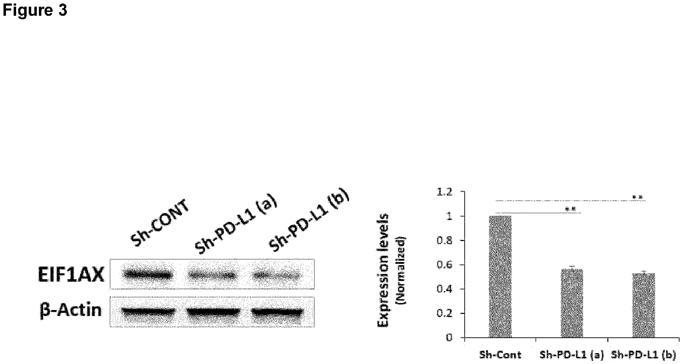

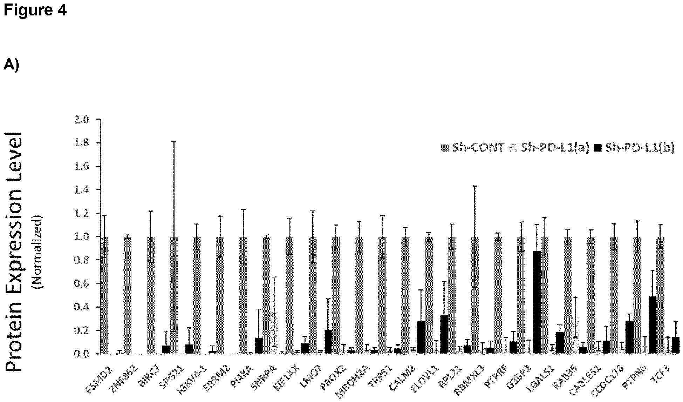

The present inventors separated the nuclear protein fraction and analyzed differentially expressed proteins (DEP) in PD-L1^{Pos} and PD-L1^{KD} MDA-MB-231 cells using SYNAPT liquid chromatography coupled with tandem mass spectrometry (LC/MS). Among the 897 proteins successfully identified, 451 proteins were DEP with a significant difference (p<0.05) between PD-L1^{Pos} and PD-L1^{KD} cells (Table 3). Further reduction using a 4-fold cut-off yielded 175 DEP between the two groups. Western blotting of a randomly selected protein (EIF1AX) from the proteomic analysis confirmed its downregulation in PD-L1^{KD} clones compared to PD-L1^{Pos} cells (Figure 3). The expression levels of the top 25-upregulated nuclear proteins in PD-L1^{Pos} cells are summarized in Figure 4A.

**Table 3. Top 25 nuclear DEP upregulated in PD-L1^{Pos} cells as compared to PD-L1^{KD} cells as shown by proteomics.**

| **Anova (P)** | **Max fold change** | **Highest mean** | **Lowest mean** | **Description** | **Gene /Protein Name** |
|---|---|---|---|---|---|
| 0.00 | Infinity | Sh-CONT | Sh-PD-L1 (a) | 26S proteasome non-ATPase regulatory subunit 2 OS=Homo sapiens GN=PSMD2 PE=1 SV=3 | **PSMD2** |
| 0.00 | Infinity | Sh-CONT | Sh-PD-L1 (b) | Zinc finger protein 862 OS=Homo sapiens GN=ZNF862 PE=2 SV=2 | ZNF862 |
| 0.01 | Infinity | Sh-CONT | Sh-PD-L1 (a) | Baculoviral IAP repeat-containing protein 730kDa subunit OS=Homo sapiens GN=BIRC7 PE=4 SV=1 | **BIRC7/Livin** |
| 0.04 | Infinity | Sh-CONT | Sh-PD-L1 (a) | Isoform 2 of Maspardin OS=Homo sapiens GN=SPG21 | SPG21 |
| 0.00 | Infinity | Sh-CONT | Sh-PD-L1 (a) | Ig kappa chain V-IV region STH (Fragment) OS=Homo sapiens PE=1 SV=1 | IGKV4-1 |
| 0.00 | Infinity | Sh-CONT | Sh-PD-L1 (b) | Serine/arginine repetitive matrix protein 2 (Fragment) OS=Homo sapiens GN=SRRM2 PE=1 SV=1 | SRRM2 |
| 0.03 | 301.1 | Sh-CONT | Sh-PD-L1 (a) | Phosphatidylinositol 4-kinase alpha OS=Homo sapiens GN=PI4KA PE=1 SV=1 | **PI4KA** |
| 0.00 | 97.0 | Sh-CONT | Sh-PD-L1 (b) | U1 small nuclear ribonucleoprotein A (Fragment) OS=Homo sapiens GN=SNRPA PE=1 SV=1 | SNRPA |
| 0.00 | 44.5 | Sh-CONT | Sh-PD-L1 (a) | Eukaryotic translation initiation factor 1A, X-chromosomal OS=Homo sapiens GN=EIF1AX PE=1 SV=1 | **EIF1AX** |
| 0.00 | 44.2 | Sh-CONT | Sh-PD-L1 (a) | LIM domain only protein 7 OS=Homo sapiens GN=LMO7 PE=1 SV=3 | LMO7/FBX20 |
| 0.01 | 31.4 | Sh-CONT | Sh-PD-L1 (b) | Isoform 2 of Prospero homeobox protein 2 OS=Homo sapiens GN=PROX2 | PROX2 |
| 0.00 | 29.0 | Sh-CONT | Sh-PD-L1 (b) | Maestro heat-like repeat-containing protein family member 2A OS=Homo sapiens GN=MROH2A PE=4 SV=2 | MROH2A |
| 0.01 | 28.8 | Sh-CONT | Sh-PD-L1 (a) | Isoform 2 of Zinc finger transcription factor Trps1 OS=Homo sapiens GN=TRPS1 | **TRPS1** |
| 0.00 | 24.2 | Sh-CONT | Sh-PD-L1 (a) | Calmodulin OS=Homo sapiens GN=CALM2 PE=1 SV=1 | **CALM2** |
| 0.02 | 23.9 | Sh-CONT | Sh-PD-L1 (a) | Elongation of very long chain fatty acids protein 1 OS=Homo sapiens GN=ELOVL1 PE=1 SV=1 | ELOVL1 |
| 0.00 | 23.4 | Sh-CONT | Sh-PD-L1 (a) | 60S ribosomal protein L21 OS=Homo sapiens GN=RPL21 PE=1 SV=1 | RPL21 |
| 0.01 | 21.8 | Sh-CONT | Sh-PD-L1 (a) | RNA-binding motif protein, X-linked-like-3 OS=Homo sapiens GN=RBMXL3 PE=2 SV=2 | RBMXL3 |
| 0.04 | 19.6 | Sh-CONT | Sh-PD-L1 (a) | Receptor-type tyrosine-protein phosphatase F OS=Homo sapiens GN=PTPRF PE=1 SV=2 | PTPRF/LAR |
| 0.01 | 18.9 | Sh-CONT | Sh-PD-L1 (a) | Isoform B of Ras GTPase-activating protein-binding protein 2 OS=Homo sapiens GN=G3BP2 | **G3BP2** |
| 0.00 | 17.7 | Sh-CONT | Sh-PD-L1 (a) | Galectin-1 OS=Homo sapiens GN=LGALS1 PE=1 SV=2 | **LGALS1/ Galectin-i (Gal1)** |
| 0.00 | 16.4 | Sh-CONT | Sh-PD-L1 (b) | Ras-related protein Rab-35 OS=Homo sapiens GN=RAB35 PE=1 SV=1 | RAB35 |
| 0.01 | 15.3 | Sh-CONT | Sh-PD-L1 (a) | Isoform 2 of CDK5 and ABL1 enzyme substrate 1 OS=Homo sapiens GN=CABLES1 | CABLES1 |
| 0.00 | 15.3 | Sh-CONT | Sh-PD-L1 (a) | Coiled-coil domain-containing protein 178 OS=Homo sapiens GN=CCDC178 PE=4 SV=1 | CCDC178 |
| 0.00 | 14.9 | Sh-CONT | Sh-PD-L1 (a) | Tyrosine-protein phosphatase nonreceptor type 6 OS=Homo sapiens GN=PTPN6 PE=1 SV=1 | PTPN6 |
| 0.03 | 14.2 | Sh-CONT | Sh-PD-L1 (a) | Isoform E47 of Transcription factor E2-alpha OS=Homo sapiens GN=TCF3 | **TCF3** |

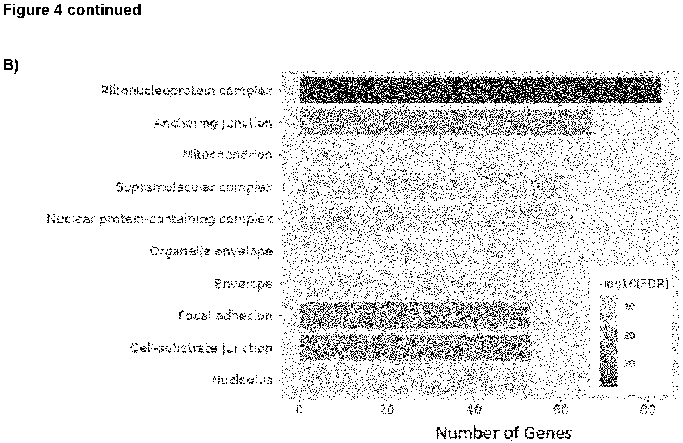

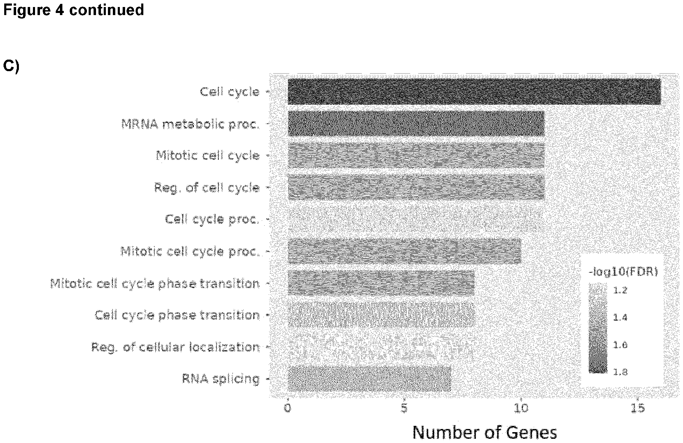

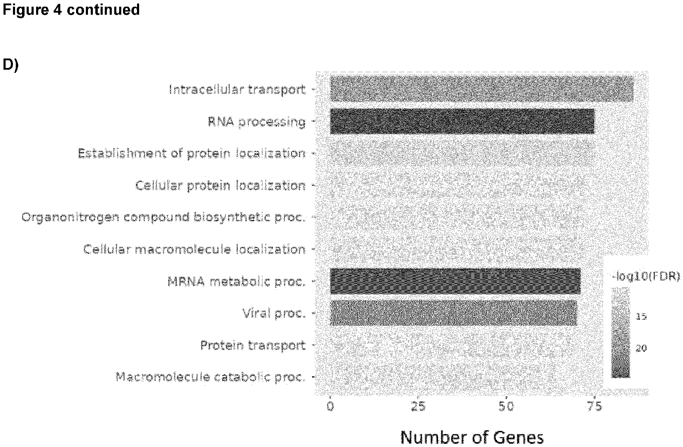

Further analysis using the graphical web-based ShinyGO Gene Enrichment application confirmed that many of the proteomics identified proteins were de facto nuclear proteins (Figure 4B). Interestingly, the upregulated proteins were mainly related to cell cycle and mitosis (Figure 4C). An in-depth literature search for the 61 overexpressed proteins in PD-L1^{Pos} cells showed that 41 were previously linked to proliferation, of which 19 (bolded) were specifically known in BC cells (Top 25 are shown in Table 3). On the other hand, 390 proteins were downregulated in PD-L1^{Pos} cells compared to their PD-L1^{KD} counterparts and ShinyGO analysis showed they were mostly related to RNA processing biology (Figure 4D). Altogether, PD-L1 enriches for nuclear proteins that have been reported to regulate cell cycle and proliferation.

### Example 3: PD-L1 modulates expression of SKP2-p21lp27 molecules through Livin and Gal1

To understand how the DEPs are related to the key proliferation, the present inventors focused on the top 25 upregulated proteins in PD-L1^{Pos} cells (Figure 4A), particularly proteins previously known to be involved in the activation of the PI3K/AKT pathway. These proteins (Table 3 - boxed in bold) were PSMD2, Livin (encoded by *BIRC7* gene), EIF1AX, CALM2, G3BP2, TCF3, and Gal1 (encoded by *LGALS1* gene). The inventors examined the transient KD effect of these proteins individually on the cell cycle regulating proteins, p27, p21 and their upstream regulator SKP2, expression using qIF. While only Livin, EIF1AX, and Gal1 gene KD significantly downregulated SKP2 expression (Figure 5A), the KD of each of these seven genes individually resulted in significant upregulation of p21 expression (Figure 5B). In contrast, only PSMD2, Livin, TCF3, and Gal1 KD significantly upregulated the expression of p27 (Figure 5C). Altogether, Livin and Gal1 KD phenotypically downregulated SKP2 and upregulated p21 and p27 (Figure 5D).

The effect of PD-L1 on Livin or Gal1 expression was further confirmed (in addition to proteomics) using qIF and western blotting. Livin was mainly nuclear and was significantly reduced in one PD-L1^{KD} clone (Sh-PD-L1(b)) as compared to its PD-L1^{Pos} counterpart (Figure 6A and B). Gal1 was expressed in the nucleus and cytoplasm, and there was a significant suppression in both cellular fractions of PD-L1^{KD} cells compared to their PD-L1^{Pos} counterparts. As nuclear Gal1 was the main fraction previously known to correlate with proliferation, its expression in the nuclear proteins was measured using western blotting (Figure 6C). Consistent with the qIF results, nuclear Gal1 expression was significantly lower in PD-L1^{KD} cells as compared with PD-L1^{Pos} cells. Similarly, transient KD of PD-L1 decreased both Gal1 and Livin expression (Figure 6D), supporting that the observed downregulation was not due to an off-target effect of PD-L1 ShRNA in PD-L1^{KD} cells. The effect of PD-L1 on Livin was confirmed in SUM159 cells. In agreement with the results seen in MDA-MB-231 cells, Livin was downregulated in both PD-L1^{KD} SUM159 clones (Figure 7), whereas Gal1 was downregulated only in one clone (data not shown).

To confirm these findings *in vivo*, the inventors used cells isolated from cryopreserved enzymedigested PD-L1^{KD} cells and their control counterparts grown as xenografts in nude mice. Livin and Gal1 levels were significantly downregulated in PD-L1^{KD} cells as compared to their PD-L1^{Pos} counterparts (Figure 6E).

### Example 4: Positive feedback loop between Livin/Gal1 and PI3KIAKT activation

Livin and Gal1, which modulate SKP2-p21/p27 axis in the examined cells, are known to be associated with PI3K/AKT signaling. To relate Livin and Gal1 to PI3K/AKT, the present inventors examined AKT phosphorylation (pAKT) following Livin and Gal1 KD in MDA-MB-231 BC cells. KD of Livin or Gal1 significantly decreased pAKT (both cytoplasmic and nuclear fractions) (Figure 8A). Similarly, suppression of the PI3K/AKT pathway using LY294002 led to a decrease in Livin and Gal1 expression (Figure 8B), suggesting a positive feedback loop between Livin/Gal1 and PI3K/AKT pathway activation.

### Example 5: Knocking down both PD-L1 and Livin provides an additive effect on inhibiting breast cancer colony formation and tumorsphere formation.

To functionally relate the above-selected proteins to cell proliferation, the colony-forming ability of MDA-MB-231 BC cells upon KD of PD-L1 and its candidate downstream effectors was assessed. PD-L1^{KD} significantly inhibited the CFA of MDA-MB-231 cells and gene KD of Livin and Gal1, in addition to EIF1AX and G3BP2, significantly decreased CFA in MDA-MB-231 BC cells. Interestingly, knocking down both PD-L1 and Livin inhibited the colony formation significantly more than each siRNA alone (Figure 9 A and B). Such a combination was not additive in RTCA (Data not shown) suggesting an effect beyond proliferation. The present inventors therefore tested the effect of this combination on tumorsphere formation, an *in vitro* assay to assess sternness and resistance of these cancer cells. Indeed, in the presence of chemotherapy, knocking down Livin and PD-L1, inhibited tumorsphere formation more than PD-L1 alone (Figure 9C).

In summary, the experiments demonstrate that PD-L1 upregulates Livin and Gal1 to activate PI3K/AKT pathway in a positive feed loop, a process that consequently promotes cancer cell proliferation and stemness. On the other hand, Livin promotes cancer cell sternness and therapy resistance (Figure 9D), thus proposing that Livin inhibition in combination with PD-L1 inhibition as a way to inhibit both proliferation and sternness of breast cancer cells.

### Conclusion

The present invention shows for the first time that PD-L1 promotes BC cell proliferation by regulating Livin and Gal1 expression, which in turn module the SKP2-p27/p21 axis, while Livin promotes cancer sternness and therapy resistance.

The present invention shows the intrinsic effect of PD-L1 on breast cancer cell proliferation and response to chemotherapy. Proteomic analysis suggested several PD-L1 downstream proteins that could promote the PI3K/AKT pathway activation through a positive feedback loop. *In vitro,* CFA functionally demonstrated the central role of Livin and Gal1 in promoting PD-L1-mediated proliferation, which is confirmed xenograft-derived cells confirmed this relationship *in vivo.*

Importantly, knocking down both PD-L1 and Livin provides an additive effect on inhibiting breast cancer colony formation more than either agents alone. In addition to this, knocking down both PD-L1 and Livin dramatically inhibits tumorsphere formation, especially in the presence of the chemotherapeutic agent, Paclitaxel.

The examples of the present invention demonstrate that PD-L1^{KD} downregulates the PI3K/AKT-associated signaling proteins Livin and Gal1 to inhibit the proliferation of BC cells. Importantly, Livin can limit cancer stem cells in addition to its effect on proliferation. Therefore, targeting Livin in combination with PD-L1 will be more effective and less toxic than AKT or PI3K inhibitors that target major pathway hubs like AKT in order to target proliferation and sternness of cancer cells.

## Claims

1. A combination of a Livin inhibitor and an inhibitor of the interaction between PD-1 and its ligand PD-L1, or a combination of a Livin inhibitor and an inhibitor of the intracellular downstream signaling pathway of a complex formed by PD-1 and PD-L1 binding, or a combination of a Livin inhibitor and an inhibitor of the intracellular downstream signaling pathway of PD-1 or PD-L1.

2. The combination according to claim 1, wherein said inhibitor of the interaction between PD-1 and its ligand PD-L1 is an inhibitor that targets PD-1 and/or an inhibitor that targets PD-L1; wherein, further, said inhibitor of the interaction between PD-1 and its ligand PD-L1 is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the interaction between PD-1 and its ligand PD-L1 is an antibody, an oligopeptide, or a small molecule inhibitor; wherein, more preferably, said inhibitor of the interaction between PD-1 and its ligand PD-L1 is an antibody; and
wherein said inhibitor of the intracellular downstream signaling pathway of a complex formed by PD-1 and PD-L1 binding is an inhibitor that targets PD-1 and/or an inhibitor that targets PD-L1 and/or an inhibitor that targets at least one member of the intracellular downstream signaling pathway of a complex formed by PD-1 and PD-L1 binding; wherein, further, said inhibitor of the intracellular downstream signaling pathway of a complex formed by PD-1 and PD-L1 binding is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the intracellular downstream signaling pathway of a complex formed by PD-1 and PD-L1 binding is an antibody, an oligopeptide, or a small molecule inhibitor; wherein, more preferably, said inhibitor of the intracellular downstream signaling pathway of a complex formed by PD-1 and PD-L1 binding is an antibody; and
wherein said inhibitor of the intracellular downstream signaling pathway of PD-1 or PD-L1 is an inhibitor that targets PD-1 or an inhibitor that targets PD-L1 and/or an inhibitor that targets at least one member of the intracellular downstream signaling pathway of PD-1 or PD-L1; wherein, further, said inhibitor of the intracellular downstream signaling pathway of PD-1 or PD-L1 is selected from a group comprising antibodies including bispecific antibodies and nanobodies, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fusion proteins, antibody prodrugs, oligopeptides, and oligonucleotides, such as aptamers, siRNAs, and shRNAs;
wherein, preferably, said inhibitor of the intracellular downstream signaling pathway of PD-1 or PD-L1 is an antibody, an oligopeptide, or a small molecule inhibitor; wherein, more preferably, said inhibitor of the intracellular downstream signaling pathway of PD-1 or PD-L1 is an antibody.

3. The combination according to any of the foregoing claims, wherein said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is selected from the group comprising Pembrolizumab, Nivolumab, Cemiplimab, Dostarlimab (TSR-042), Retifanlimab, Camrelizumab (SHR1210), Sintilimab (IBI308), Toripalimab (JS 001) , Tislelizumab (BGB-A317), Spartalizumab (PDR001), AMP-224, AMP-514 (MEDI0680), Acrixolimab (YBL-006), REGN2810, Prolgolimab (BCD-100), PF-0681591, Ezabenlimab (BI-754091), Geptanolimab (GB226), Zimberelimab (GLS-010), LZM009, Pucotenlimab (HX008), Atezolizumab, Avelumab, Durvalumab (Medi4736), Cosibelimab, Envafolimab (KN035), CA-170, AUNP12, BMS-986189, M7824, Pacmilimab (CX-072), FAZ-053, LY-3300054, SHR-1316, and Socazolimab (ZKAB001).

4. The combination according to any of the foregoing claims, wherein said Livin inhibitor is selected from a group comprising oligopeptides, small molecule inhibitors, antibodies including bispecific antibodies and nanobodies, vaccines, oligonucleotides, such as aptamers, siRNAs, shRNAs, and nucleic acids such as DNA and RNA, encoding said oligopeptides, antibodies including bispecific antibodies and nanobodies, vaccines, and oligonucleotides, such as aptamers, siRNAs, and shRNAs;
wherein, preferably, said Livin inhibitor is selected from a group comprising oligopeptides, small molecule inhibitors, antibodies, and vaccines;
wherein, more preferably, said Livin inhibitor is an oligopeptide or a small molecule inhibitor.

5. The combination according to any of the foregoing claims, wherein said Livin inhibitor is selected from IAP-inhibitors, wherein, preferably, said IAP-inhibitors are selected from a group comprising JQ1, SMAC mimetics such as AZD5582, SM-164, Birinapant (TL32711), GDC-0152, LCL161, and BV6, and a vaccine targeting Livin.

6. The combination according to any of the foregoing claims, wherein said Livin inhibitor is selected from a group comprising JQ1, BDB oligopeptides, Livin-binding peptides, and vaccines targeting Livin.

7. The combination according to any of the foregoing claims, wherein said combination comprises one or more further agent(s) or drug(s), such as chemotherapeutic drugs;
optionally, wherein said chemotherapeutic drugs are selected from a group comprising Paclitaxel, Docetaxel, Epiribicin, Eribulin, Doxorubicin, Cyclophosphamide, Methotrexate, Fluorouracil, Capecitabine, Carboplatin, Gemcitabine, Nab-paclitaxel, and Vinorelbine; wherein, preferably, said chemotherapeutic drug is Paclitaxel.

8. A combination as defined in any of claims 1 to 7, for use in a method of treatment of cancer; wherein, preferably, said cancer is selected from: breast cancer, hepatocellular carcinoma, melanoma, oesophageal cancer, renal cancer, colon cancer, colorectal cancer, lung cancer, mesothelioma, bladder cancer, gastrointestinal cancer, head and neck squamous cell cancer including nasopharyngeal carcinoma, Hodgkin's lymphoma, gastric/gastroesophageal junction (GEJ) adenocarcinoma, endometrial carcinoma, primary mediastinal large B-cell lymphoma, Merkel cell carcinoma, urothelial cancer, ovarian cancer, and prostate cancer;
wherein, more preferably, said cancer is breast cancer;
optionally, wherein said combination is administered to a patient in need thereof;
wherein, preferably, said patient is a mammal; wherein, more preferably, said patient is a human.

9. The combination for use according to claim 8, wherein said breast cancer is selected from: triple-negative breast cancer, locally advanced non-inflammatory breast cancer, invasive ductal carcinoma, inflammatory breast cancer, metastatic breast cancer, medullary carcinoma, tubular carcinoma, mucinous carcinoma, adenoid cystic carcinoma of the breast, metaplastic breast cancer, basal type breast cancer, and papillary breast cancer;
optionally, wherein the cells of said breast cancer contain mutations in the BRCA1 and/or BRCA2 gene.

10. The combination for use according to any of claims 8 to 9, wherein cells of said breast cancer are **characterized by** being estrogen receptor-negative, or progesterone receptor-negative, or human epidermal growth factor receptor 2-negative, or any possible combination thereof; wherein, preferably, cells of the cancer are **characterized by** a combination thereof; wherein, even more preferably, cells of the cancer are **characterized by** being estrogen receptor-negative, progesterone receptor-negative, and human epidermal growth factor receptor 2-negative, also called triple-negative breast cancer.

11. The combination for use according to any of claims 8 to 10, wherein in said method, administering said combination to said patient in need thereof comprises the steps:
(a) administering a dose of said Livin inhibitor to said patient, and
(b) administering a dose of said inhibitor of the interaction between PD-1 and its ligand PD-L1 to said patient; or administering a dose of said inhibitor of the intracellular downstream signaling pathway of a complex formed by PD-1 and PD-L1 binding to said patient; or administering a dose of said inhibitor of the intracellular downstream signaling pathway of PD-1 or PD-L1 to said patient;
wherein step (a) is performed before step (b), or step (b) is performed before step (a), or step (a) and step (b) are performed simultaneously;
further wherein steps (a) and (b) are performed once or wherein steps (a) and (b) are repeated in periodic cycles.

12. The combination for use according to any of claims 8 to 11, wherein said combination is administered to a patient in need thereof for a treatment duration ranging from 1 day to lifetime; preferably 1 day to 30 years; more preferably 1 day to 10 years; even more preferably 1 day to 5 years; even more preferably 1 day to 2 years; even more preferably 1 day to 12 months; most preferably 1 day to 6 months.

13. The combination for use according to any of claims 8 to 12, wherein, in said method of treatment, said combination for use is administered in a pharmaceutical composition, said pharmaceutical composition comprising pharmaceutically acceptable excipient(s) and/or carrier(s);
optionally, wherein said combination is supplemented to an ongoing therapy for treatment of breast cancer.

14. The combination for use according to any of claims 8 to 13, wherein said Livin inhibitor is formulated and administered as an injection intravenously or subcutaneously, a pill, a tablet, a capsule, a suppository, an oral solution, a buccal, a granule, a powder, a suspension, a solution, a spray, an ointment, drops, or patches; preferably an injection intravenously or subcutaneously;
wherein, further, said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is formulated and administered as an injection intravenously or subcutaneously, a pill, a tablet, a capsule, a suppository, an oral solution, a buccal, a granule, a powder, a suspension, a solution, a spray, an ointment, drops, or patches; preferably an injection intravenously or subcutaneously.

15. A kit for the preparation of an infusion solution for the administration of a combination of a Livin inhibitor and an inhibitor of the interaction between PD-1 and its ligand PD-L1, or a kit for the preparation of an infusion solution for the administration of a combination of a Livin inhibitor and an inhibitor of the intracellular downstream signaling pathway of a complex formed by PD-1 and PD-L1 binding, or a kit for the preparation of an infusion solution for the administration of a combination of a Livin inhibitor and an inhibitor of the intracellular downstream signaling pathway of PD-1 or PD-L1, as defined in any of claims 1 to 7 and/or 8 to 14, said kit comprising either:
(A) a first container containing a composition comprising a Livin inhibitor, and a second container containing a composition comprising a pharmaceutically acceptable diluent; and
a third container containing a composition comprising an inhibitor of the interaction between PD-1 and its ligand PD-L1, or an inhibitor of the intracellular downstream signaling pathway of a complex formed by PD-1 and PD-L1 binding, or an inhibitor of the intracellular downstream signaling pathway of PD-1 or PD-L1, and a fourth container containing a composition comprising a pharmaceutically acceptable diluent;
or said kit comprising:
(B) a first container containing said Livin inhibitor pre-mixed with said inhibitor of the interaction between PD-1 and its ligand PD-L1; or a first container containing said Livin inhibitor pre-mixed with said inhibitor of the intracellular downstream signaling pathway of a complex formed by PD-1 and PD-L1 binding; or a first container containing said Livin inhibitor pre-mixed with said inhibitor of the intracellular downstream signaling pathway of PD-1 or PD-L1; and a second container containing a composition comprising a pharmaceutically acceptable diluent;
wherein said containers are preferably selected from vials, bottles, pre-filled syringes, ampoules, and infusion bags.

16. The kit according to claim 15, wherein said Livin inhibitor is provided in a form selected from: an injection concentrate, a pre-diluted solution, and a lyophilized powder for reconstitution;
wherein said inhibitor of the interaction between PD-1 and its ligand PD-L1, or said inhibitor of the intracellular downstream signaling pathway of a complex formed by PD-1 and PD-L1 binding, or said inhibitor of the intracellular downstream signaling pathway of PD-1 or PD-L1 is provided in a form selected from: an injection concentrate, a pre-diluted solution, and a lyophilized powder for reconstitution.
